# EUROPÄISCHE PATENTSCHRIFT

(11) **EP 2 047 249 B1**
(45) Veröffentlichungstag und Bekanntmachung des Hinweises auf die Patenterteilung: **03.06.2015**
(21) Anmeldenummer: 07785681.3
(22) Anmeldetag: 27.07.2007
(51) Int. Cl.: G01N 33/18, G01N 33/24

(54) **THERMOKATALYTISCHES KOHLENWASSERSTOFFMESSGERÄT ZUR LECKDETEKTION AN UNTERSEEPIPELINES**
DEVICE FOR RECORDING MEASUREMENT DATA
DISPOSITIF POUR SAISIR DES DONNÉES DE MESURE

(30) Priorität: 28.07.2006 DE 102006035788
(43) Veröffentlichungstag der Anmeldung: 15.04.2009
(73) Patentinhaber: Contros Systems&Solutions GmbH, 24148 Kiel (DE)
(72) Erfinder: ESSER, Daniel, 22527 Hamburg (DE)
(74) Vertreter: Patentanwälte Klickow & Partner Partnerschaftsgesellschaft mbB
(86) Internationale Anmeldenummer: PCT/DE2007/001373
(87) Internationale Veröffentlichungsnummer: WO 2008/011881

(56) Entgegenhaltungen:
- EP-A- 0 823 055
- GARCIA, MASSON: "Environmental and geologic application of solid-state methane sensors" ENVIRONMENTAL GEOLOGY, Bd. 46, 2004, Seiten 1059-1063, XP002465252
- SANDBERG C ET AL: "The application of a continuous leak detection system to pipelines and associated equipment" PROC. OF PCIC 1988, 12. September 1988 (1988-09-12), Seiten 241-244, XP010078417
- MELLIN T A ET AL: "Autonomous Underwater System for Pipeline Leak Detection and Inspection" PROC. OF THE 6TH INTL. SYMP. ON UNMANNED UNTETHERED SUBMERSIBLE TECHNOLOGY, 12. Juni 1989 (1989-06-12), Seiten 15-24, XP010325340

## Beschreibung

Die Erfindung betrifft eine Vorrichtung zur Erfassung von Messdaten, die ein Gehäuse, einen Gehäuseinnenraum sowie eine vom Gehäuse gehalterte und den Gehäuseinnenraum bereichsweise begrenzende Membran aufweist und bei der innerhalb des Gehäuseinnenraumes mindestens ein Sensor angeordnet ist sowie bei der im Gehäuseinnenraum ein thermokatalytisches Element zur Zersetzung mindestens eines gasförmigen Kohlenwasserstoffes angeordnet ist und bei der der Gehäuseinnenraum mit einer Gestaltung zur Unterstützung einer Gaszirkulation ausgebildet ist.

Derartige Vorrichtungen werden beispielsweise verwendet, um eine meßtechnische Erfassung von Gasen durchzuführen, die durch die Membran hindurchtreten. Die Membran schirmt hierbei zum einen den Sensor gegenüber einer Umgebung ab, darüber hinaus sorgt die Membran dafür, dass nur vordefinierte Gase in nennenswerter Konzentration in den Bereich des Sensors gelangen können. Derartige Sensoranordnungen werden beispielsweise in der EP 0 823 055 B1 sowie der EP 1 114 297 B1 beschrieben.

Ein Problem bei der Verwendung derartiger Sensoren bzw. Messanordnungen besteht darin, dass die Gase, die durch die Membran hindurch in die Umgebung des Sensors gelangt sind, nur relativ langsam auch wieder aus dem Bereich des Sensors entweichen, wenn sich die Gaskonzentration in der Umgebung der Messanordnung verändert. Bei sich zeitlich ändernden Konzentrationen der zu erfassenden Gaskonzentrationen treten hierdurch nicht unerhebliche Zeitkonstanten auf, die zu einer meßtechnischen Trägheit der Gesamtanordnung führen.

Bei einer Bewegung durch ein örtliches Messgebiet mit unterschiedlichen zu messenden Konzentrationen bzw. bei zeitlich veränderlichen zu messenden Konzentrationen können die bekannten Messanordnungen deshalb noch nicht alle Anforderungen erfüllen, die an eine optimale Messqualität gestellt werden.

In GARCIA, MASSON: "Environmental and geologic application of solid-state methane sensors", ENVIRONMENTAL GEOLOGY, Bd. 46, 2004, Seiten 1059-1063 wird eine Vorrichtung zur Erfassung von Messdaten beschrieben, die ein Gehäuse und einen Gehäuseinnenraum aufweist. Vom Gehäuse wird eine Membran gehaltert und innerhalb des Gehäuseinnenraumes ist ein Sensor angeordent. Der Sensor ist als ein thermokatalytisches Element ausgebildet.

Aufgabe der vorliegenden Erfindung ist es, eine Vorrichtung der einleitend genannten Art derart zu verbessern, dass eine vergrößerte Messdynamik bereitgestellt wird.

Diese Aufgabe wird erfindungsgemäß dadurch gelöst, dass im Bereich des Gehäuses eine Temperaturstabilisierung angeordnet ist und dass im Bereich des Gehäuseinnenraumes mindestens eine Pumpe angeordnet ist.

Durch die Anordnung eines thermokatalytischen Elementes innerhalb des Gehäuseinnenraumes ist es möglich, störende Gaskonzentrationen kurzfristig erheblich zu minimieren. Dies können entweder Gase sein, die zusätzlich zum zu messenden Gas durch die Membran hindurchtreten und ansonsten das Meßergebnis verfälschen würden, insbesondere kann aber auch das zu messende Gas selbst thermokatalytisch zersetzt werden, um eine hochdynamische Messung mit geringen Trägheitseffekten zu unterstützen. Als thermokatalytische Elemente können insbesondere thermokatalytische Sensoren, sogenannten Pellistoren, zum Einsatz kommen.

Die erfindungsgemäße Meßanordnung eignet sich beispielsweise dazu, Leckagen im Bereich von Offshore-Pipelines nachzuweisen. Aufgrund der sehr schnellen Ansprechzeiten ist eine Verwendung im Bereich von Unterwasserfahrzeugen möglich. Es ergibt sich eine sehr große Langzeitstabilität, sodaß die Meßanordnung auch für Langzeiteinsätze für einen Zeitraum von einem Jahr oder länger geeignet ist. Bei Verwendung von optischen Sensoren ergibt sich eine Langzeitstabilität von bis zu zehn Jahren.

Die Gaszirkulation wird aktiv durch die Pumpe generiert und kann Zusätzlich als Nebeneffekt der thermokatalytischen Zersetzung erzeugt werden. Die thermokatalytische Zersetzung führt in der Regel zu einer lokalen Gaserwärmung, die zu Bewegungen des innerhalb des Gehäuseinnenraum eingeschlossenen Gases verursacht. Insbesondere durch eine geeignete Gestaltung der Wandungen des Gehäuseinnenraumes können lokale Gasströmungen oder Gasturbulenzen generiert werden, die einen Kontakt der in den Gehäuseinnenraum durch die Membran hindurch eindringenden Kohlenwasserstoffe mit dem thermokatalytischen Sensor unterstützen.

Eine Anwendung im Bereich der Mineralölwirtschaft wird dadurch unterstützt, daß das thermokatalytische Element zur Zersetzung mindestens eines Kohlenwasserstoffes ausgebildet ist.

Offshore-Anwendungen werden dadurch ermöglicht, daß das Gehäuse zur Verwendung im Wasser ausgebildet ist.

Leckageerfassungen im Bereich von Pipelines werden dadurch unterstützt, daß die Meßanordnung zur Erfassung von Gasen im Bereich von Wasser ausgebildet ist.

Eine äußerst hohe Meßgenauigkeit kann dadurch erreicht werden, daß der Sensor als ein optischer Sensor ausgebildet ist.

Zur Unterstützung eines geringen Energieverbrauches wird vorgeschlagen, daß der Sensor als ein Halbleitersensor ausgebildet ist.

Eine zweckmäßige Ausführung der Membran besteht darin, daß die Membran gasdurchlässig ausgebildet ist.

Eine einfache fertigungstechnische Realisierung wird dadurch unterstützt, daß die Membran als eine Beschichtung ausgebildet ist.

Zu einem kompakten Aufbau trägt es bei, daß die Membran auf dem Sensor angeordnet ist.

Eine hohe Druckstabilität kann dadurch bereitgestellt werden, daß die Membran auf einem gasdurchlässigen Träger angeordnet ist.

Eine hohe Druckbeständigkeit bei gleichzeitig geringem Strömungswiderstand für das zu messende Gas kann dadurch erreicht werden, daß der Träger aus einem porösem Material ausgebildet ist.

Zu einer hohen Meßdynamik trägt es bei, daß die Membran bidirektional gasdurchlässig ausgebildet ist.

Kurze Ansprechzeiten werden auch dadurch unterstützt, daß außenseitig am Gehäuse eine Pumpe angeordnet ist.

Lang andauernde Verwendungen im Unterwasserbereich werden dadurch unterstützt, daß die Membran Antifouling-Eigenschaften aufweist.

Die Züchnungen zeigen :
Fig. 1 einen schematischen Querschnitt durch eine nicht erfindungsgemäße Meßanordnung,
Fig. 2 eine schematische Darstellung zur Veranschaulichung des Funktionsprinzips und
Fig. 3 eine detailliertere prinzipielle Darstellung der erfindungsgemäßen Meßanordnung mit zugeordneten elektrischen Komponenten.

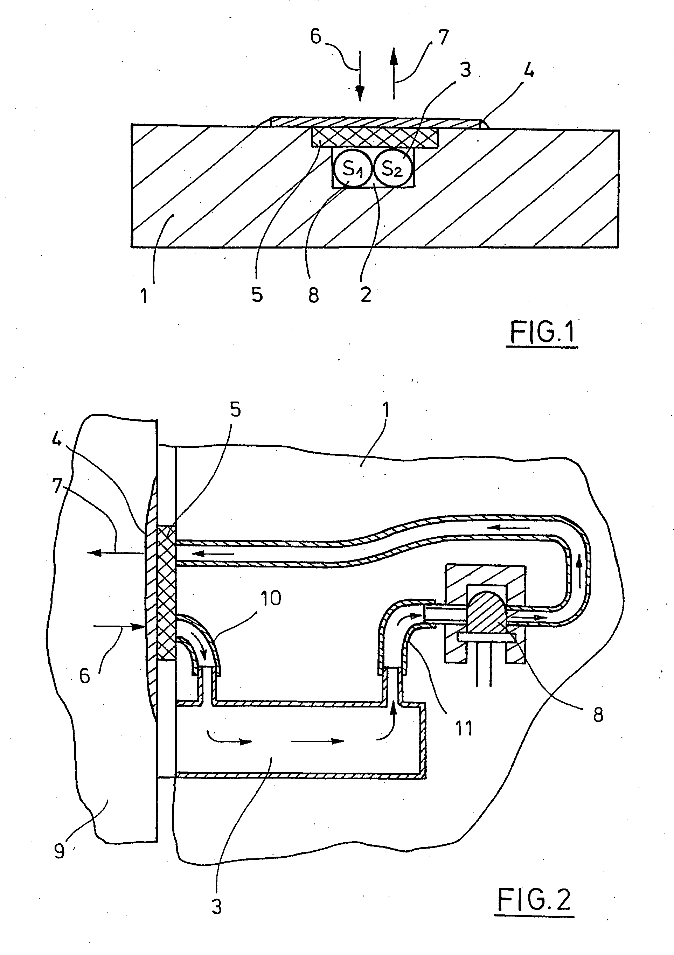

Gemäß Fig. 1 besteht eine Meßanordnung aus einem Gehäuse (1), das mit einem Gehäuseinnenraum (2) versehen ist. Im Bereich des Gehäuseinnenraumes ist ein Sensor (3) angeordnet. Der Gehäuseinnenraum (2) ist bereichsweise von einer Membran (4) begrenzt, die vom Gehäuse (1) gehaltert ist. Die Membran kann als klassische Membran oder in Art einer Beschichtung ausgebildet sein. Beim dargestellten Ausführungsbeispiel erstreckt sich die Membran bereichsweise über einen Träger (5), um eine vergrößerte mechanische Stabilität bereitzustellen. Der Träger (5) kann aus beliebigen gasdurchlässigen Materialien ausgebildet sein. Insbesondere ist an die Verwendung eines porösen Materials gedacht.

Die Membran (4) ist bidirektional gasdurchlässig, so daß eine Gasströmung sowohl in einer Einströmrichtung (6) als auch in einer Ausströmrichtung (7) erfolgen kann.

Der Sensor (3) kann beispielsweise zum Nachweis von Kohlenwasserstoffen oder anderen Gasen ausgebildet sein. Gedacht ist beispielsweise an die Messung von Methan, Butan oder Propan. Es können aber grundsätzlich aber auch beliebige andere physikalische, chemische oder biologische Parameter unter Verwendung des Sensors (3) erfaßt werden.

Im Geräteinnenraum (2) und benachbart zum Sensor (3) ist zusätzlich ein thermokatalytisches Element (8) angeordnet. Das thermokatalytische Element (8) kann beispielsweise als ein thermokatalytischer Sensor ausgebildet sein. Ein thermokatalytischer Sensor führt in der Regel die Verbrennung einer zu zersetzenden Substanz durch. Beispielsweise ist es denkbar, Methan unter Zusatz von Sauerstoff thermokatalytisch in Kohlendioxid und Wasser zu zersetzen.

Fig. 2 veranschaulicht eine Verwendung der Meßanordnung. Innerhalb des Gehäuses (1) ist hier eine Gaszirkulation vorgesehen, die beispielsweise durch mindestens eine nicht dargestellte Pumpe unterstützt wird. Aus einem externen Medium (9), beispielsweise Wasser, durch die Membran (4) hindurchtretendes Gas wird über eine Leitung (10) mit dem Sensor (3) zugeführt und durchströmt diesen. Über eine Leitung (11) wird das Gas anschließend am thermokatalytischen Element (8) vorbeigeführt und tritt wieder durch die Membran (4) aus. Der Anteil am meßtechnisch interessierenden Gas wird unter Verwendung des thermokatalytischen Elementes (8) hierbei derart herabgesetzt, daß das austretende Gas das in Einströmrichtung (6) eintretende Gas hinsichtlich der interessierenden Konzentrationen nicht nennenswert verfälscht.

Als Sensor (3) können beispielsweise optische Sensoren oder Halbleitersensoren verwendet werden. Unter einer Membran (4) werden sowohl eigentliche Membranen als auch Beschichtungen mit membranartigen Eigenschaften verstanden.

Als Material für die Membran (4) können Silikone oder silikonartige Substanzen zum Einsatz kommen, grundsätzlich erweisen sich aber auch eine Vielzahl anderer Materialien als brauchbar. Die Membran (4) hat typischer Weise sowohl die Funktion einer selektiven Zuführung des zu messenden Gases zum Sensor (3) als auch die Funktion eines Schutzes des Sensors (3) vor eindringendem Wasser bei einer Verwendung im Unterwasserbereich. Es können auch Membranvarianten verwendet werden, die alle im Wasser enthaltenen Gase durchlassen. Der Träger (5) erweist sich insbesondere bei einer Verwendung der Meßanordnung in größeren Wassertiefen als vorteilhaft, da die mechanische Stabilität der Membran (4) erheblich verbessert wird. Die Konstruktion ermöglicht Anwendungen in einer Wassertiefe von bis zu 6000 Metern.

Unter Verwendung der Meßanordnung ist es möglich, sowohl im Wasser gelöste Gase als auch beispielsweise in Form von Gasblasen oder durch Anlagerung im Wasser enthaltene Gase meßtechnisch zu erfassen. Die Meßanordnung kann auch im Sediment bzw. dem Meeresgrund eingeführt werden und hier Gase, beilspielsweise Kohlenwasserstoffe, meßtechnisch erfassen. Neben der bereits erwähnten meßtechnischen Erfassung von Kohlenwasserstoffen wie Methan, Ethan, Propan und Butan können durch Auswahl geeigneter Sensoren (3) auch andere Gase, beispielsweise Kohlendioxid oder Schwefelwasserstoff, meßtechnisch erfaßt werden.

Grundsätzlich ergeben sich eine Vielzahl von Einsatzbereichen. Beispielhaft werden die folgenden Einsatzbereiche genannt: Leckagedetektion an Offshore-Pipelines, Messungen an Unterwasservulkanen, Messungen an hydrothermalen Vents, Messung in Kläranlagen von Mülldeponien, Methanaustritte bei Tunnelbohrungen für Straßen, Messung von Methan in Bohrlöchern, allgemeine Messungen in der Ozeanographie, Methan im Wattenmeer, Biogasanlagen, Offhore-Sicherheit an Bohr- und Förderplattformen, Herstellung von Methanhydraten im Labor, Exploration von Erdgas- und Erdöllagerstätten, Detektion von Grundwasseraustritten in Küstennähe, Methanmessungen in Seen und Methangasquellen in Kanälen (Marine Seeps).

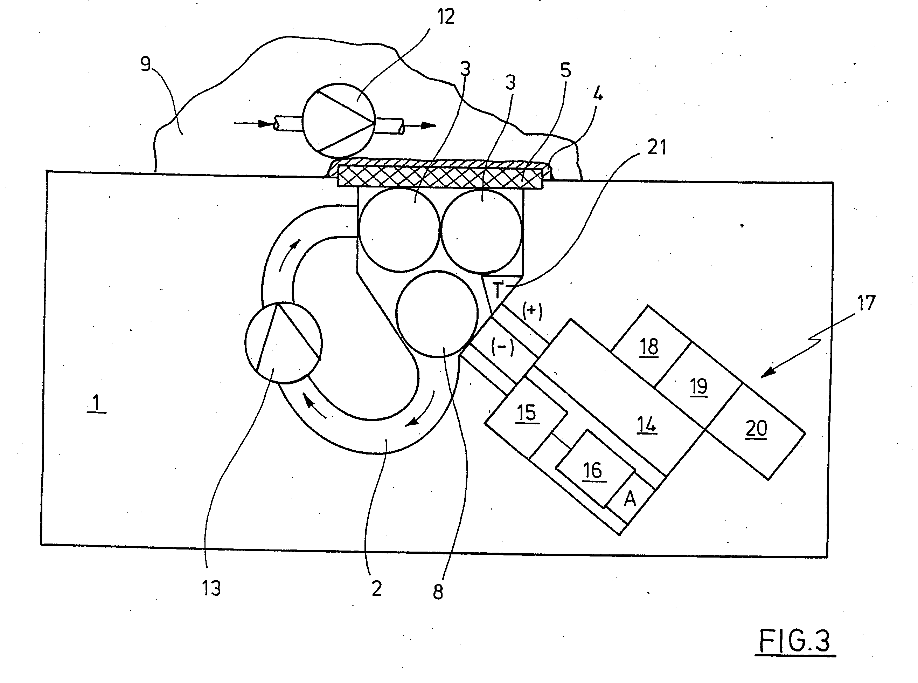

Fig. 3 zeigt eine detailliertere konstruktive Realisierung der erfindungsgemäßen Meßanordnung. Im Gehäuseinnenraum (2) sind hier zwei Sensoren (3) angeordnet. Der erste Sensor (3) ist als Halbleitergassensor zum Nachweis mindestens eines Gases ausgebildet. Der zweite Sensor (3) ist als ein Infrarotsensor insbesondere im Wellenbereich NDIR ausgebildet, um mindestens ein Gas zu detektieren. Als thermokatalytisches Element (8) wird ein als Pellistor realisierter thermokatalytischer Sensor verwendet, der zum Nachweis von Kohlenwasserstoffen geeignet ist und insbesondere eine Verbrennung von Kohlenwasserstoffen durchführen kann.

Außenseitig am Gehäuse (1) ist eine Pumpe (12) angeordnet, die bei einer Realisierung des externen Mediums (9) als Wasser als Wasserpumpe ausgebildet ist. Die Pumpe (12) erzeugt eine Strömung des Wassers und damit auch der im Wasser gelösten Gase in Richtung auf die Membran (4) und verursacht hierdurch Turbulenzen im Bereich der außenseitigen Begrenzung der Membran (4), die zu einer verstärkten Desorption des Gases durch die Membran (4) führen. Der Gehäuseinnenraum (2) ist mit einer Pumpe (13) ausgestattet, um eine Gaszirkulation zu unterstützen und hierdurch die Meßeffektivität weiter zu erhöhen.

Die Meßanordnung ist mit einer Energieversorgung (14) versehen. Zur Unterstützung mobiler Anwendungen ist die Energieversorgung (14) beispielsweise als Batterie oder Akkumulator realisiert. Gemäß einer typischen Ausführungsform sind der oder die Sensoren (3) als analoge Sensoren ausgebildet, deren Ausgangssignal einem oder mehreren Analog-Digital-Wandlern (15) zugeführt wird, die das oder die Meßsignale in digital weiterverarbeitbare Signale transformieren. Der Analog-Digital-Wandler (15) kann beispielsweise mit einem Datenspeicher (16) verbunden sein, um die Durchführung der Messung zu dokumentieren und/oder eine zeitlich versetzte Datenauswertung zu unterstützen.

Die Meßanordnung ist ebenfalls mit einer Steuereinheit (17) versehen, die mit einem Speicher (18), einer Kontrolleinheit (19) sowie einer Schnittstelle (20) ausgestattet ist. Der Speicher (18) kann beispielsweise als Flash-ROM ausgebildet sein. Als Kontrolleinheit (19) kann ein Mini-PC zum Einsatz kommen, der einen oder mehrere Mikroprozessoren sowie sonstige elektronische Bauelemente aufweist. Die Schnittstelle (20) dient insbesondere zur Durchführung einer Datenübertragung, wahlweise online und/oder offline.

Zur weiteren Verbesserung der Meßqualität trägt es bei, im Bereich des Gehäuseinnenraumes (2) eine Temperaturstabilisierung (21) anzuordnen. Die Temperaturstabilisierung (21) verhindert im Bereich von feuchten Anwendungen insbesondere ein Unterschreiten des Taupunktes. Darüber hinaus würden aber auch größere Temperaturänderungen auf die Meßgenauigkeit ungünstige Auswirkungen haben.

## Patentansprüche

1. Vorrichtung zur Erfassung von Messdaten, die ein Gehäuse (1), einen Gehäuseinnenraum (2) sowie eine vom Gehäuse gehalterte und den Gehäuseinnenraum (2) bereichsweise begrenzende Membran (4) aufweist und bei der innerhalb des Gehäuseinnenraumes mindestens ein Sensor (3) angeordnet ist, sowie bei der im Gehäuseinnenraum (2) ein thermokatalytisches Element (8) zur Zersetzung mindestens eines gasförmigen Kohlenwasserstoffes angeordnet ist und bei der der Gehäuseinnenraum (2) mit einer Gestaltung zur Unterstützung einer Gaszirkulation ausgebildet ist, **dadurch gekennzeichnet, dass** im Bereich des Gehäuses (1) eine Temperaturstabilisierung (21) angeordnet ist und dass im Bereich des Gehäuseinnenraumes (2) mindestens eine Pumpe (13) angeordnet ist, und dass der Sensor eingerichtet ist, eine Konzentration mindestens eines gasförmigen Stoffes messtechnisch zu erfassen.

2. Vorrichtung nach Anspruch 1, **dadurch gekennzeichnet, dass** das Gehäuse (1) zur Verwendung im Wasser ausgebildet ist.

3. Vorrichtung nach einem der Ansprüche 1 oder 2, **dadurch gekennzeichnet, dass** die Messanordnung zur Erfassung von Gasen im Bereich von Wasser ausgebildet ist.

4. Vorrichtung nach einem der Ansprüche 1 bis 3, **dadurch gekennzeichnet, dass** der Sensor (3) als ein optischer Sensor ausgebildet ist.

5. Vorrichtung nach einem der Ansprüche 1 bis 3, **dadurch gekennzeichnet, dass** der Sensor (3) als ein Halbleitersensor ausgebildet ist.

6. Vorrichtung nach einem der Ansprüche 1 bis 5, **dadurch gekennzeichnet, dass** die Membran (4) gasdurchlässig ausgebildet ist.

7. Vorrichtung nach einem der Ansprüche 1 bis 6, **dadurch gekennzeichnet, dass** die Membran (4) als eine Beschichtung ausgebildet ist.

8. Vorrichtung nach einem der Ansprüche 1 bis 7, **dadurch gekennzeichnet, dass** die Membran (4) auf dem Sensor (3) angeordnet ist.

9. Vorrichtung nach einem der Ansprüche 1 bis 7, **dadurch gekennzeichnet, dass** die Membran (4) auf einem gasdurchlässigen Träger (5) angeordnet ist.

10. Vorrichtung nach Anspruch 9, **dadurch gekennzeichnet, dass** der Träger (5) aus einem porösem Material ausgebildet ist.

11. Vorrichtung nach einem der Ansprüche 1 bis 10, **dadurch gekennzeichnet, dass** die Membran (4) bidirektional gasdurchlässig ausgebildet ist.

12. Vorrichtung nach einem der Ansprüche 1 bis 11, **dadurch gekennzeichnet, dass** außenseitig am Gehäuse (1) eine Pumpe angeordnet ist.

13. Vorrichtung nach einem der Ansprüche 1 bis 12, **dadurch gekennzeichnet, dass** die Membran (4) Antifouling-Eigenschaften aufweist.

## Claims

1. Device for recording measurement data, which has a housing (1), a housing inner chamber (2), and a membrane (4) which is supported by the housing and delimits part of the housing inner chamber (2) and in which at least one sensor (3) is arranged inside the housing inner chamber, and in which a thermocatalytic element (8) for decomposing at least one gaseous hydrocarbon is arranged in the housing inner chamber (2), and in which the housing inner chamber (2) has a configuration which facilitates gas circulation, **characterised in that** a temperature stabiliser (21) is arranged in the region of the housing (1) and **in that** at least one pump (13) is arranged in the region of the housing inner chamber (2), and **in that** the sensor is configured to record a concentration of at least one gaseous substance by measurement technology.

2. Device according to claim 1, **characterised in that** the housing (1) is designed for use in water.

3. Device according to either claim 1 or claim 2, **characterised in that** the measurement arrangement is designed for determining gases in water.

4. Device according to any one of claims 1 to 3, **characterised in that** the sensor (3) is in the form of an optical sensor.

5. Device according to any one of claims 1 to 3, **characterised in that** the sensor (3) is in the form of a semiconductor sensor.

6. Device according to any one of claims 1 to 5, **characterised in that** the membrane (4) is designed to be gas-permeable.

7. Device according to any one of claims 1 to 6, **characterised in that** the membrane (4) is in the form of a coating.

8. Device according to any one of claims 1 to 7, **characterised in that** the membrane (4) is arranged on the sensor (3).

9. Device according to any one of claims 1 to 7, **characterised in that** the membrane (4) is arranged on a gas-permeable carrier (5).

10. Device according to claim 9, **characterised in that** the carrier (5) is made of a porous material.

11. Device according to any one of claims 1 to 10, **characterised in that** the membrane (4) is designed to be gas-permeable in two directions.

12. Device according to any one of claims 1 to 11, **characterised in that** a pump is arranged outside the housing (1).

13. Device according to any one of claims 1 to 12, **characterised in that** the membrane (4) has antifouling properties.

## Revendications

1. Dispositif pour la saisie de données, qui comprend un boîtier (1), un espace intérieur de boîtier (2) ainsi qu'une membrane (4), qui est maintenue par le boîtier et limite, par endroits, l'espace intérieur (2) du boîtier, et ledit dispositif étant doté d'au moins un détecteur (3), agencé dans l'espace intérieur (2) du boîtier, ainsi qu'un élément thermo catalytique (8) qui, logé dans l'espace intérieur (2) du boîtier, est destiné à décomposer au moins un carbure d'hydrogène à l'état gazeux, et l'espace intérieur (2) du boîtier étant conçu pour assister une circulation de gaz,
**caractérisé en ce que**, dans la région du boîtier (1), est agencé un système de stabilisation de température (21) et q u e, dans la région de l'espace intérieur (2) du boîtier, est installée une pompe (13), et q u e le détecteur est aménagé pour détecter, selon la méthode de mesure technique, une concentration d'au moins une substance gazeuse.

2. Dispositif selon la revendication 1,
**caractérisé en ce que** le boîtier (1) est conçu pour être utilisé dans l'eau.

3. Dispositif selon revendication 1 ou 2,
**caractérisé en ce que** le système de mesure pour la détection de gaz est réalisé dans le région de l'eau.

4. Dispositif selon l'une des revendications 1 à 3,
**caractérisé en ce que** le détecteur (3) est réalisé en tant que détecteur optique.

5. Dispositif selon l'une des revendications 1 à 3,
**caractérisé en ce que** le détecteur (3) est réalisé en tant que détecteur semiconducteur.

6. Dispositif selon l'une des revendications 1 à 5,
**caractérisé en ce que** la membrane (4) est de conception perméable au gaz.

7. Dispositif selon l'une des revendications 1 à 6,
**caractérisé en ce que** la membrane (4) est réalisée sous la forme d'un revêtement.

8. Dispositif selon l'une des revendications 1 à 7,
**caractérisé en ce qu**e la membrane (4) est disposée sur le détecteur (3).

9. Dispositif selon l'une des revendications 1 à 7,
**caractérisé en ce que** la membrane (4) est disposée sur un support (5) perméable au gaz.

10. Dispositif selon la revendication 9,
**caractérisé en ce que** le support (5) est formé à partir d'un matériau poreux.

11. Dispositif selon l'une des revendications 1 à 10,
**caractérisé en ce que** la membrane (4) est perméable au gaz dans deux directions.

12. Dispositif selon l'une des revendications 1 à 11,
**caractérisé en ce que**, côté extérieur, une pompe est aménagée sur le boîtier (1).

13. Dispositif selon l'une des revendications 1 à 12,
**caractérisé en ce que** la membrane (4) est de dotée de propriétés anti pourriture.
